# EUROPÄISCHE PATENTSCHRIFT

(11) **EP 1 689 735 B1**
(45) Veröffentlichungstag und Bekanntmachung des Hinweises auf die Patenterteilung: **16.01.2008**
(21) Anmeldenummer: 04764403.4
(22) Anmeldetag: 24.08.2004
(51) Int. Cl.: C07D 317/00

(54) **VERFAHREN ZUR HERSTELLUNG VON GLYCERINCARBONATMETHACRYLAT**
METHOD FOR PRODUCING GLYCEROL CARBONATE METHACRYLATE
PROCEDE POUR PRODUIRE DU METHACRYLATE DE CARBONATE DE GLYCEROL

(30) Priorität: 26.11.2003 DE 10355830
(43) Veröffentlichungstag der Anmeldung: 16.08.2006
(73) Patentinhaber: Evonik Röhm GmbH, 64293 Darmstadt (DE)
(72) Erfinder: SCHMITT, Bardo, 55252 Mainz (DE); CASPARI, Maik, 64665 Alsbach-Hähnlein (DE)
(86) Internationale Anmeldenummer: PCT/EP2004/009423
(87) Internationale Veröffentlichungsnummer: WO 2005/058862

(56) Entgegenhaltungen:
- EP-A- 0 001 088
- EP-A- 0 236 994
- EP-A- 0 328 150
- EP-A- 0 837 049
- EP-A- 0 908 905
- EP-A- 1 201 640
- WO-A-03/022796
- DE-A1- 3 937 116
- FR-A- 2 539 740
- FR-A- 2 747 596
- US-A- 2 979 514
- US-A- 4 202 990
- US-A- 4 423 235
- US-A- 4 882 391
- US-A- 5 374 699
- PATENT ABSTRACTS OF JAPAN Bd. 2003, Nr. 12, 5. Dezember 2003 (2003-12-05) & JP 2003 327854 A (JAPAN PAINT MANUFACTURERS ASSOCIATION), 19. November 2003 (2003-11-19)
- PATENT ABSTRACTS OF JAPAN Bd. 2000, Nr. 05, 14. September 2000 (2000-09-14) & JP 2000 040526 A (MITSUI CHEMICALS INC.), 8. Februar 2000 (2000-02-08)
- PATENT ABSTRACTS OF JAPAN & JP 03 156803 A (FUJI PHOTO FILM CO LTD), 4. Juli 1991 (1991-07-04)

## Beschreibung

Die Erfindung betrifft ein Verfahren zur Herstellung von Glycerincarbonatmethacrylat in Gegenwart von Metall-Chelat-Katalysatoren vom Typ Metallion-1,3-Diketonat, insbesondere Zirkonacetylacetonat.

In der Lackindustrie ist (2-Oxo-1,3-dioxolan-4-yl)methylmethacrylat (Glycerincarbonatmethacrylat) ein viel verwendeter Vernetzer. Es sind verschiedene Herstellverfahren für Glycerincarbonatmethacrylat bekannt.

In JP 2001018729 wird Glycerincarbonat mit Acrylsäurechlorid umgesetzt. Die anfallenden Chlorid-Abfälle stellen eine große Umweltbelastung dar. In WO 2000031195 wird Glycidylmethacrylat mit CO₂ umgesetzt. Dieses Verfahren wird unter hohem Druck durchgeführt. Die dafür notwendige Verfahrensapparatur ist aufwendig und teuer. In DE 3937116 wird ein cyclocarbonathaltiger Alkohol mit einer Carbonsäure bei erhöhter Temperatur und in Gegenwart eines sauren Katalysators umgesetzt. Das gewünschte Produkt wird nach Destillation in einer Reinheit von 75,5% erhalten. Die Ausbeute variiert in Abhängigkeit von der verwendeten Säure zwischen 25,5 und 83%. Mit der hier erzielten Reinheit von nur 75,5% kann das Produkt in vielen Anwendungen nicht verwendet werden.

Aufgabe der Erfindung war es Glycerincarbonatmethacrylat in hoher Reinheit und mit hohen Ausbeuten herzustellen.

Die Aufgabe wurde gelöst durch ein Verfahren zur Herstellung von (2-Oxo-1,3-dioxolan-4-yl)methylmethacrylat, bei dem Methylmethacrylat in Gegenwart von Stabilisatoren und eines Metall-Chelat-Katalysators vom Typ Metallion-1,3-Diketonat, insbesondere Zirkonacetylacetonat mit Glycerincarbonat umgeestert wird.

Überraschend wurde gefunden, dass durch den Einsatz von Zirkonacetylacetonat als Katalysator unter sehr milden Bedingungen gearbeitet werden kann. Die Umesterung in Gegenwart von Zirkonacetylacetonat erfolgt bei 50-80°C, bevorzugt bei 70°C.

Zirkonacetylacetonat wird bevorzugt in Mengen von 0,1-5,0 Gew.-%, besonders bevorzugt von von 1,0-3,0 Gew.-%, bezogen auf das Gesamtgewicht des Ansatzes, eingesetzt.

Als Katalysator können neben Zirkonacetylacetonat auch andere Metall-1,3-Diketonate, wie z.B. Lithium- oder Zink-1,3-Diketonate oder 1,3-Diphenylpropan-1,3-dion verwendet werden.

Es wurde gefunden, dass durch die erfindungsgemäße Reaktionsführung ein niedriger Vernetzergehalt erzielt wird. Ein niedriger Vernetzergehalt wirkt sich in Anwendungen dadurch aus, dass verbesserte mechanische Eigenschaften bei der Copolymerisation mit anderen monofunktionellen Monomeren erzielt werden. Das Material ist weniger spröde. Als Vernetzer werden Glycerindimethacrylat und Glycerintrimethacrylat beobachtet. Vorzugsweise wird im Produkt ein Gehalt von weniger als 5-Gew.-%, besonders bevorzugt von weniger als 3 Gew.-%, Vernetzer beobachtet.

Außerdem wurde gefunden, dass das Produkt mit hohen Ausbeuten und in hoher Reinheit hergestellt werden kann. Es werden Ausbeuten über 80% erzielt, mit Reinheiten des Produktes von ca. 90%. Durch die erzielten Reinheiten kann auf eine aufwendige destillative Reinigung verzichtet werden. Das Monomer besitzt einen hohen Siedepunkt und ist daher nur im Hochvakuum abtrennbar. Es entfällt eine aufwendige Destillationsapparatur und die Gefahr der Polymerisation durch die hohe thermische Belastung, was bei diesem Monomer häufig beobachtet wird.

Bei der Herstellung von (2-Oxo-1,3-dioxolan-4-yl)methylmethacrylat werden Stabilisatoren zugefügt, die eine radikalische Polymerisation der (Meth)acrylgruppen während der Reaktion verhindern. Diese Stabilisatoren sind in der Fachwelt weithin bekannt.

Eingesetzt werden hauptsächlich 1,4-Dihydroxybenzole. Es können jedoch auch anders substituierte Dihydroxybenzole zum Einsatz kommen. Allgemein lassen sich derartige Stabilisatoren mit der allgemeinen Formel (1) wiedergeben 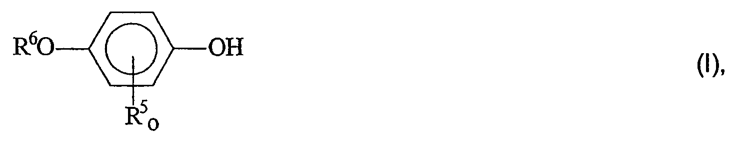 worin
R⁵ einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br;
o eine ganze Zahl im Bereich von eins bis vier, vorzugsweise eins oder zwei ist; und
R⁶ Wasserstoff, einen linearen oder verzweigten Alkylrest mit eins bis acht Kohlenstoffatomen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl oder tert.-Butyl.

Es können jedoch auch Verbindungen mit 1,4-Benzochinon als Stammverbindung eingesetzt werden. Diese lassen sich mit der Formel (11) beschreiben 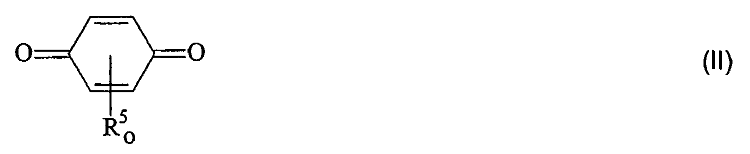 worin
R⁵ einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br; und
o eine ganze Zahl im Bereich von eins bis vier, vorzugsweise eins oder zwei ist.

Ebenso werden Phenole der allgemeinen Struktur (III) eingesetzt 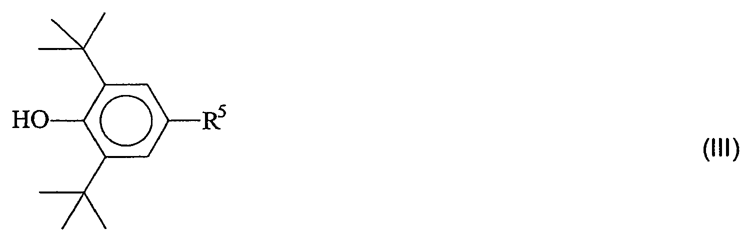 worin
R⁵ einen linearen oder verzweigten Alkylrest mit eins bis acht Kohlenstoffatomen, Aryl oder Aralkyl, Propionsäureester mit 1 bis 4 wertigen Alkoholen, welche auch Heteroatome wie S, O und N enthalten können, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl bedeutet.

Eine weitere vorteilhafte Substanzklasse stellen gehinderte Phenole auf Basis von Triazinderivaten der Formel (IV) dar 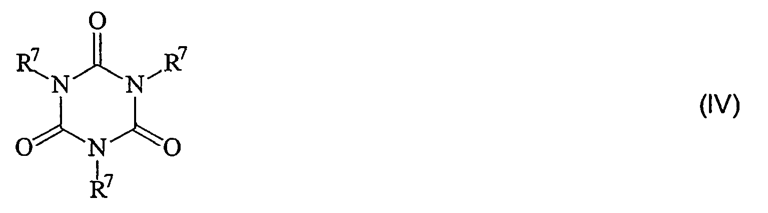 mit R⁷ = Verbindung der Formel (V) 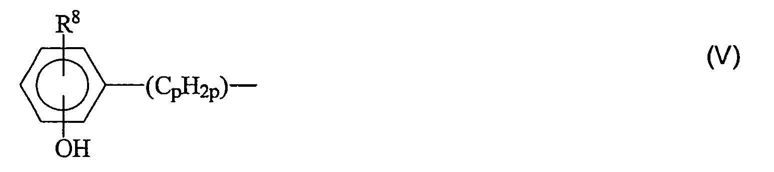 worin
R⁸ = CₚH₂ₚ-₁
mit p = 1 oder 2 ist.

Besonders erfolgreich werden die Verbindungen 1,4-Dihydroxybenzol, 4-Methoxyphenol, 2,5-Dichloro-3,6-dihydroxy-1,4-benzochinon, 1,3,5-Trimethyl-2,4,6-tris-(3,5-di-tert.butyl-4-hydroxybenzyl)benzol, 2,6-Di-tert. butyl-4-methylphenol, 2,4-Dimethyl-6-tert. butylphenol, 2,2-Bis [3,5-Bis(1,1-dimethylethyl)-4-hydroxyphenyl-1-oxopropoxymethyl)]1,3-propandiylester, 2,2'-Thiodiethylbis-[3-(3,5-di-tert.butyl-4-hydroxyphenyl)]propionat, Octadecyl-3-(3,5-di-tert.butyl-4-hydroxyphenyl)propionat, 3,5-Bis(1,1-dimethylethyl-2,2-Methylenbis-(4-methyl-6-tert.butyl)phenol, Tris-(4-tert.butyl-3-hydroxy-2,6-dimethylbenzyl)-s-triazin-2,4,6-(1H,3H,5H)trion, Tris (3,5-ditert.butyl-4-hydroxy)-s-triazin-2,46-(1H, 3H,5H) trion oder tert Butyl-3,5-dihydroxybenzol, besonders bevorzugt 4-Hydroxy-2,2,6,6-tetramethylpiperidinooxyl, Hydrochinon, 4-Methyl-2,6-di-tert-butylphenol, Hydrochinonmonomethylether, 2-tert-Butyl-6-(3-tert-butyl-2-hydroxy-5-methylbenzyl)-4-methylphenylacrylat, 4-(Methacryloyloxy)-2,2,6,6-tetramethylpiperidin-1-oxyl oder 2,5-Di-tert-butylhydrochinon, eingesetzt.

Im Rahmen der Erfindung wird eine Tocopherolverbindung zur Stabilisierung von ethylenisch ungesättigten Monomeren verwendet.

Bei den im Sinne der Erfindung verwendbaren Tocopherolverbindungen handelt es sich um in 2-Stellung mit einem 4,8,12-Trimethyltridecyl-Rest substituierte Chroman-6-ole (3,4-Dihydro-2*H-*1-benzopyran-6-ole). Zu den erfindungsgemäß vorzugsweise einsetzbaren Tocopherolen gehören alpha-Tocopherol, beta-Tocopherol, gamma-Tocopherol, delta-Tocopherol, zeta2-Tocopherol und eta-Tocopherol, alle der vorgenannten Verbindungen jeweils in der (2R, 4'R,8'R)-Form, sowie alpha-Tocopherol in der (all-rac)-Form. Bevorzugt sind alpha-Tocopherol in der (2R, 4'R,8'R)-Form (Trivialname: RRR-alpha-Tocopherol) sowie das synthetische racemische alpha-Tocopherol (all-rac-alpha-Tocopherol). Hiervon wiederum ist letztgenanntes aufgrund des relativ niedrigen Preises besonders interessant.

Bezogen auf das Gewicht der gesamten Reaktionsmischung beträgt der Anteil der Stabilisatoren einzeln oder als Mischung im allgemeinen 0,01 - 0,50 Gew.-%, wobei man die Konzentration der Stabilisatoren vorzugsweise so auswählt, dass die Farbzahl gemäß DIN 55945 nicht beeinträchtigt wird. Viele dieser Stabilisatoren sind kommerziell erhältlich.

Glycerincarbonatmethacrylat kann als funktionelles Monomer in Copolymerisaten von Lacken und Klebstoffen verwendet werden, mit dem eine nachträgliche, polymeranaloge Reaktion, unter anderem die Vernetzung mit difunktionellen Aminen in einer Lackformulierung, möglich wird. Außerdem kann es in Batterie-Elektrolyten, Extrusionsharzen und zur Metallextraktion verwendet werden.

Die im Folgenden gegebenen Beispiele werden zur besseren Veranschaulichung der vorliegenden Erfindung gegeben, sind jedoch nicht dazu geeignet, die Erfindung auf die hierin offenbarten Merkmale zu beschränken.

### Beispiele

### Beispiel 1

118 g (1,0 mol) Glycerincarbonat werden mit 600 g (6,0 mol) Methylmethacrylat und 0,14 g 4-Hydroxy-2,2,6,6-tetramethylpiperidinooxyl (Tempol) in einem Rundkolben mit Destillationsapparatur erwärmt. Eventuell vorhandenes Wasser wird azeotrop mit Methylmethacrylat abdestilliert. Anschließend wird die Mischung leicht abgekühlt. 18,0 g Zirkonacetylacetonat und die dem Azeotropdestillat äquivalente Menge Methylmethacrylat werden der Mischung zugefügt. Das Gemisch wird zum Sieden erhitzt. Die Alkoholyse wird bei 70°C Kopftemperatur begonnen. Gegen Ende der Reaktion erhöht sie sich auf 100°C.

Nach Ende der Alkoholyse wird die Mischung abgekühlt und der Katalysator Zirkonacetylacetonat mit verdünnter Phosphorsäure gefällt. Anschließend wird die Suspension über einen Druckfilter gegeben und das Filtrat abgetrennt.

Zur Abtrennung des Glycerincarbonats wird das Filtrat im Scheidetrichter mit einer verdünnten NaCl Lösung ausgeschüttelt. Das Filtrat wird am Rotationsverdampfer bei 70°C und 200-10 mbar entgast.

Die Ausbeute beträgt 80,6%. Die Reinheit des Produktes 91,4%.
Versuchsnummer: B1

### Beispiel 2

236 g (2,0 mol) Glycerincarbonat werden mit 1200 g (12,0 mol) Methylmethacrylat und 0,29 g 4-Hydroxy-2,2,6,6-tetramethylpiperidinooxyl (Tempol) in einem Rundkolben mit Destillationsapparatur erwärmt. Eventuell vorhandenes Wasser wird azeotrop mit Methylmethacrylat abdestilliert. Anschließend wird die Mischung leicht abgekühlt. 28,7 g Zirkonacetylacetonat und die dem Azeotropdestillat äquivalente Menge Methylmethacrylat werden der Mischung zugefügt. Das Gemisch wird zum Sieden erhitzt. Die Alkoholyse wird bei 70°C Kopftemperatur begonnen. Gegen Ende der Reaktion erhöht sie sich auf 100°C.

Nach Ende der Alkoholyse wird die Mischung abgekühlt und der Katalysator Zirkonacetylacetonat mit verdünnter Phosphorsäure gefällt. Anschließend wird die Suspension über einen Druckfilter gegeben und das Filtrat abgetrennt.

Zur Abtrennung des Glycerincarbonats wird das Filtrat im Scheidetrichter mit verdünnter NaCl Lösung ausgeschüttelt. Das Filtrat wird am Rotationsverdampfer bei 70°C und 200-10 mbar entgast.

Die Ausbeute beträgt 87,4%. Die Reinheit des Produktes 89,7%.
Versuchsnummer: B2

### Vergleichsbeispiel 1

### lsopropyltitanat

236 g (2;0 mol) Glycerincarbonat werden mit 600 g (6,0 mol) Methylmethacrylat und 0,14 g 4-Hydroxy-2,2,6,6-tetramethylpiperidinooxyl (Tempol) in einem Rundkolben mit Destillationsapparatur erwärmt. Eventuell vorhandenes Wasser wird azeotrop mit Methylmethacrylat abdestilliert. Anschließend wird die Mischung leicht abgekühlt. 8,4 g lsopropyltitanat und die dem Azeotropdestillat äquivalente Menge Methylmethacrylat werden der Mischung zugefügt. Das Gemisch wird zum Sieden erhitzt. Die Alkoholyse wird bei 70°C Kopftemperatur begonnen. Die Reaktion ist aber sehr träge und wird deshalb nach 3 Stunden abgebrochen und überschüssiges Methylmethacrylat (MMA) abdestilliert. Der Rohester wird analytisch untersucht.
Versuchsnummer: V1

### Vergleichsbeispiel 2

### Dioctylzinnoxid

118 g (1,0 mol) Glycerincarbonat werden mit 600 g (6,0 mol) Methylmethacrylat und 0,14 g 4-Hydroxy-2,2,6,6-tetramethylpiperidinooxyl (Tempol) in einem Rundkolben mit Destillationsapparatur erwärmt. Eventuell vorhandenes Wasser wird azeotrop mit Methylmethacrylat abdestilliert. Anschließend wird die Mischung leicht abgekühlt. 14,4 g Dioctylzinnoxid und die dem Azeotropdestillat äquivalente Menge Methylmethacrylat werden der Mischung zugefügt. Das Gemisch wird zum Sieden erhitzt. Die Reaktion startet aber nicht, es bildet sich kein Methanol und der Versuch wird abgebrochen.
Versuchsnummer: V2

### Vergleichsbeispiel 3

### LiOH/CaO

118 g (1,0 mol) Glycerincarbonat werden mit 600 g (6,0 mol) Methylmethacrylat und 0,14 g 4-Hydroxy-2,2,6,6-tetramethylpiperidinooxyl (Tempol) in einem Rundkolben mit Destillationsapparatur erwärmt. Eventuell vorhandenes Wasser wird azeotrop mit Methylmethacrylat abdestilliert. Anschließend wird die Mischung leicht abgekühlt. 4,0 g LiOH, 10,4 g CaO und die dem Azeotropdestillat äquivalente Menge Methylmethacrylat werden der Mischung zugefügt. Das Gemisch wird zum Sieden erhitzt. Die Alkoholyse wird bei 70 °C Kopftemperatur begonnen. Gegen Ende der Reaktion erhöht sie sich auf 100 °C.

Anschließend wird der Ansatz abgekühlt, über einen Druckfilter gegeben und das Filtrat abgetrennt. Zur Abtrennung des Glycerincarbonats wird das Filtrat im Scheidetrichter mit verdünnter NaCl Lösung ausgeschüttelt. Das Filtrat wird am Rotationsverdampfer bei 70°C und 200-10 mbar entgast.

Die Ausbeute beträgt 79,6%. Die Reinheit des Produktes 52,7%.
Versuchsnummer: V3

### Vergleichsbeispiel 4

### Lithiummethylat

118 g (1,0 mol) Glycerincarbonat werden mit 600 g (6,0 mol) Methylmethacrylat und 0,14 g 4-Hydroxy-2,2,6,6-tetramethylpiperidinooxyl (Tempol) in einem Rundkolben mit Destillationsapparatur erwärmt. Eventuell vorhandenes Wasser wird azeotrop mit Methylmethacrylat abdestilliert. Anschließend wird die Mischung leicht abgekühlt. 14,4 g Lithiummethylat und die dem Azeotropdestillat äquivalente Menge Methylmethacrylat werden der Mischung zugefügt. Das Gemisch wird zum Sieden erhitzt. Die Alkoholyse wird bei 70 °C Kopftemperatur begonnen. Gegen Ende der Reaktion erhöht sie sich diese auf 100 °C. Nach Beendigung der Reaktion wird überschüssiges MMA abdestilliert. Der Rohester wird filtriert und anschließend analytisch untersucht.
Versuchsnummer: V4

Die Versuchsergebnisse werden in der nachfolgenden Tabelle zusammengefasst:

| **Versuch** | **Katalysator** | **Rohester** | | | | | **Monomer rein** | | | | |
|---|---|---|---|---|---|---|---|---|---|---|---|
| | | **(MMA haltig)** | | | | | | **GC : *** | | | |
| | Menge auf Ansatz in % | Alkohol | Produkt | HS1 | HS2 | Polytest in MeOH | Ausbeute | Alkohol | Produkt | HS1 | HS2 |
| | | FI % | FI % | FI % | FI % | | % d. Th. | FI % | FI % | FI % | FI % |
| B1 | Zirkon acetylacetonat | 8,2 | 70,1 | 0,8 | 0,6 | klar | 80,6 | 0,9 | 91,4 | 0,9 | 0,9 |
| | 2,5 | | | | | | | | | | |
| B2 | Zirkon acetylacetonat | 8,1 | 72,7 | 0,9 | 0,9 | klar | 87,4 | 0,8 | 89,7 | 1,0 | 1,3 |
| | 2,0 | | | | | | | | | | |
| V1 | Isopropyltitanat | 90,7 | 6,5 | n. b. | n. b. | n. b. | n. b. | n. b. | n. b. | n. b. | n. b. |
| | 1,0 | | | | | | | | | | |
| V2 | Dioktylzinnoxid# | n.b. | n.b. | n. b. | n. b. | n. b. | n. b. | n. b. | n. b. | n. b. | n. b. |
| | 2,0 | | | | | | | | | | |
| V3 | LiOH/CaO | 7,5 | 49,5 | 12 | 25 | klar | 79,6 | 0,3 | 52,7 | 12,3 | 27,3 |
| | 2,0 | | | | | | | | | | |
| V4 | Lithiummethylat | 2,2 | 68,9 | 1,6 | 20 | n. b. | n. b. | n. b. | n. b. | n. b. | n. b. |
| | 2,5 | | | | | | | | | | |

| | | | | | | | | | | | |
|---|---|---|---|---|---|---|---|---|---|---|---|
| Legende: HS 1 Glycerindimethacrylat # kein Umsatz GC: weitere Peaks nicht * berücksichtigt HS 2 Glycerintrimethacrylat n.b. nicht bestimmt | | | | | | | | | | | |

## Patentansprüche

1. Verfahren zur Herstellung von (2-Oxo-1,3-dioxolan-4.-yl)methyl-methacrylat, **dadurch gekennzeichnet, dass** Methylmethacrylat in Gegenwart von Stabilisatoren und eines Metall-Chelat-Katalysators vom Typ Metallion-1,3-Diketonat mit Glycerincarbonat umgeestert wird.

2. Verfahren zur Herstellung von (2-Oxo-1,3-diöxolan-4-yl)methylmethacrylat, **dadurch gekennzeichnet, dass** der Katalysator Zirkonacetylacetonat ist.

3. Verfahren zur Herstellung von (2-Oxo-1,3-dioxolan-4-yl)methyl-methacrylat, **dadurch gekennzeichnet, dass** die Umesterung bei 50-80°C erfolgt.

4. Verfahren zur Herstellung von (2-Oxo-1,3-dioxolan-4-yl)methyl-methacrylat, **dadurch gekennzeichnet, dass** die Umesterung bei 70°C erfolgt.

5. Verfahren zur Herstellung von (2-Oxo-1,3-dioxolan-4-yl)methylmethacrylat, **dadurch gekennzeichnet, dass** Zirkonacetylacetonat in Mengen von 0,1-5,0 Gew.-%, bezogen auf das Gesamtgewicht des Ansatzes, eingesetzt wird.

6. Verfahren zur Herstellung von (2-Oxo-1,3-dioxolan-4-yl)methylmethacrylat, **dadurch gekennzeichnet, dass** Zirkonacetylacetonat in Mengen von 1,0-3,0 Gew.-%, bezogen auf das Gesamtgewicht des Ansatzes, eingesetzt wird.

7. Verfahren zur Herstellung von (2-Oxo-1,3-dioxolan-4-yl)methylmethacrylat, **dadurch gekennzeichnet, dass** der während der Herstellung gebildete Vernetzergehalt weniger als 5 Gew.-%, insbesondere weniger als 3 Gew.-% beträgt.

8. Verfahren zur Herstellung von (2-Oxo-1,3-dioxolan-4-yl)methylmethacrylat, **dadurch gekennzeichnet, dass** Stabilisatoren in Mengen von 0,01-0,50 Gew.-% eingesetzt werden.

## Claims

1. Process for preparing (2-oxo-1,3-dioxolan-4-yl)-methyl methacrylate, **characterized in that** methyl methacrylate is transesterified with glycerol carbonate in the presence of stabilizers and a metal chelate catalyst of the metal ion 1,3-diketonate type.

2. Process for preparing (2-oxo-1,3-dioxolan-4-yl)-methyl methacrylate, **characterized in that** the catalyst is zirconium acetylacetonate.

3. Process for preparing (2-oxo-1,3-dioxolan-4-yl)-methyl methacrylate, **characterized in that** the transesterification takes place at 50-80°C.

4. Process for preparing (2-oxo-1,3-dioxolan-4-yl)-methyl methacrylate, **characterized in that** the transesterification takes place at 70°C.

5. Process for preparing (2-oxo-1,3-dioxolan-4-yl)-methyl methacrylate, **characterized in that** zirconium acetylacetonate is used in amounts of 0.1-5.0% by weight, based on the total weight of the batch.

6. Process for preparing (2-oxo-1,3-dioxolan-4-yl)-methyl methacrylate, **characterized in that** zirconium acetylacetonate is used in amounts of 1.0-3.0% by weight, based on the total weight of the batch.

7. Process for preparing (2-oxo-1,3-dioxolan-4-yl)-methyl methacrylate, **characterized in that** the amount of crosslinker formed during the preparation is less than 5% by weight, in particular less than 3% by weight.

8. Process for preparing (2-oxo-1,3-dioxolan-4-yl)-methyl methacrylate, **characterized in that** stabilizers are used in amounts of 0.01-0.50% by weight.

## Revendications

1. Procédé de préparation du méthacrylate de (2-oxo-1,3-dioxolann-4-yl)méthyle, **caractérisé en ce qu'**on transestérifie avec du carbonate de glycérol du méthacrylate de méthyle en présence de stabilisants et d'un catalyseur chélate métallique du type 1,3-dicétonate d'ion métallique.

2. Procédé de préparation du méthacrylate de (2-oxo-1,3-dioxolann-4-yl)méthyle, **caractérisé en ce que** le catalyseur est l'acétylacétonate de zirconium.

3. Procédé de préparation du méthacrylate de (2-oxo-1,3-dioxolann-4-yl)méthyle, **caractérisé en ce que** la transestérification est effectuée à 50-80°C.

4. Procédé de préparation du méthacrylate de (2-oxo-1,3-dioxolann-4-yl)méthyle, **caractérisé en ce que** la transestérification est effectuée à 70°C.

5. Procédé de préparation du méthacrylate de (2-oxo-1,3-dioxolann-4-yl)méthyle, **caractérisé en ce que** l'acétylacétonate de zirconium est utilisé en des quantités de 0,1 à 5,0 % en poids par rapport au poids total de la masse réactionnelle.

6. Procédé de préparation du méthacrylate de (2-oxo-1,3-dioxolann-4-yl)méthyle, **caractérisé en ce que** l'acétylacétonate de zirconium est utilisé en des quantités de 1,0 à 3,0 % en poids par rapport au poids total de la masse réactionnelle.

7. Procédé de préparation du méthacrylate de (2-oxo-1,3-dioxolann-4-yl)méthyle, **caractérisé en ce que** la teneur en agent de réticulation formé au cours de la préparation est inférieure à 5 % en poids, en particulier inférieure à 3 % en poids.

8. Procédé de préparation du méthacrylate de (2-oxo-1,3-dioxolann-4-yl)méthyle, **caractérisé en ce que** les stabilisants sont utilisés en des quantités de 0,01 à 0,50 % en poids.
